# EUROPEAN PATENT APPLICATION

(11) **EP 2 796 158 A1**
(43) Date of publication of application: **29.10.2014**
(21) Application number: 13164761.2
(22) Date of filing: 22.04.2013
(51) Int. Cl.: A61M 5/14, H05B 33/08

(54) **Apparatus including light sources**

(71) Applicant: Sanofi-Aventis Deutschland GmbH, 65929 Frankfurt am Main (DE)
(72) Inventor: Prager, Roman, 2230 Gänserndorf (AT); Hoffmann, Christian, 3100 St. Pölten (AT)

(57) **Abstract**

Apparatus comprises a first light source (11); and a second light source (12) connected in series with the first light source (11) between first and second supply terminals. A controllable switch (18) is coupled in parallel across the first light source (11). A controller (14) is configured to selectively control the controllable switch (18) to conduct, thereby selectively switching the first light source (11) on and off whilst the second light source (12) remains switched on.

## Description

### Field of the Invention

This invention relates to apparatus including first and second light sources, in particular connected in series with one another.

### Background to the Invention

Some applications require multiple light sources, and require the relative brightnesses of those light sources to have a pre-defined relationship. For instance, the brightnesses might be required to be the same. However, manufacturing tolerances may be such that devices of the same model and produced in the same batch by a manufacturing process may not have the same brightness for a given energisation. For example, different light-emitting diodes (LEDs) from a batch of the same model or device type may produce different light output intensities for a given energising current signal. For many applications, this is not a problem.

For applications where the relative brightnesses of the light sources has an effect on device operation, it can be required to provide a separate energising supply to each light source. An example of an application where equal brightnesses for LEDs may be required is in a supplemental or clip-on device for attachment to an injection device, such as Sanofi's SoloStar ™ insulin injection pen. Such a device is shown and described in WO 2011/117212, although details of the illumination arrangement are not provided.

A supplemental device may in the future use multiple LEDs to illuminate a number sleeve of the injection pen, allowing reading by a camera of numerals that are present within a dosage window of the injection pen at a given instant. Improved evenness of illumination can be provided where the LEDs have the same light intensity output. An uneven illumination of the number sleeve may result in less reliable reading of a number present in the dosage window of the injection pen or may require overillumination, reducing power efficiency.

Where separate control of multiple light sources is required, one possible solution is to provide each with a separate controllable current sink or current source. By allowing the current through each light source to be controlled, the light intensity output of the current sources can be controlled to be the same as one another. Such an arrangement would normally be provided with a DC to DC converter to reduce power consumption of the circuit.

### Summary of the Invention

A first aspect of the invention provides apparatus comprising at least two light sources including:
a first light source;
at least one second light source connected in series with the first light source between first and second supply terminals;
a controllable switch coupled in parallel across the first light source; and
a controller,
wherein the controller is configured to selectively control the controllable switch to conduct, thereby selectively switching the first light source on and off whilst the second light source remains switched on.

This can provide a required balance of illumination by the light sources whilst allowing them to be connected in series, requiring only one device for energising the light sources. This can provide effective operation whilst requiring less hardware than alternative arrangements.

The apparatus may comprise a current sink connected in series with the first and second light sources between the first and second supply terminals. This can energise both light sources together, except when the controllable switch is caused to conduct. The current sink may be located between the light sources and negative supply voltage, although it could instead be located between the light sources or between the positive supply voltage and the light sources.

The apparatus may comprise a second controllable switch coupled in parallel across the second light source, wherein the controller is configured to selectively control the second controllable switch to conduct, thereby selectively switching the second light source on and off whilst the first light source remains switched on. This can allow the apparatus to switch either light source off, depending on requirements.

The apparatus may comprise one or more further light sources connected in series with the first and second light sources between the first and second supply terminals. Each further light source may be provided with a further controllable switch coupled in parallel across the further light source, and the controller may be configured to selectively control the further controllable switch to conduct, thereby selectively switching the further light source on and off whilst the other light sources remains switched on.

The or each controllable switch may be a transistor, or it may be an electro-mechanical switch, or it may be an analogue current bypass device, for instance.

The controller may be configured to selectively control a controllable switch to conduct by applying a pulse width modulated signal to the controllable switch. Here, a duty cycle of the pulse width modulated signal may be derived from calibration data stored in a memory of the apparatus.

Alternatively, the controller may be configured to selectively control a controllable switch to conduct by applying a signal of a specific duration to the controllable switch. Here, the specific duration may be derived from calibration data stored in a memory of the apparatus.

The light sources may be light-emitting diodes, but they may alternatively be any other kind of light source in which brightness is dependent on current.

The light sources may be the same model of device, or they might differ.

The invention also provides a supplemental device for mating to an injection device, the supplemental device comprising apparatus as above, in which the light sources illuminate a number sleeve of the injection device in use.

### Brief Description of the Drawings

Embodiments of the invention will now be described, by way of example only, with reference to the accompanying drawings, in which:
Figure 1 is a circuit diagram of apparatus according to the present invention; and
Figure 2 is a timing diagram showing signals provided by a controller of the Figure 1 apparatus according to first and second embodiments of the invention.

### Detailed Description of the Embodiments

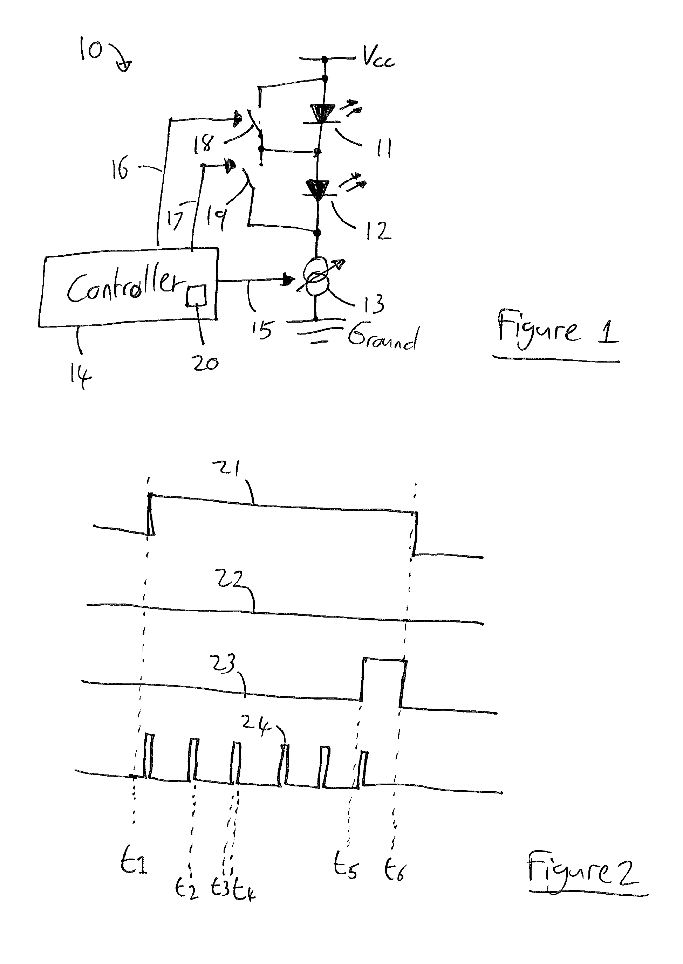

Referring firstly to Figure 1, apparatus 10 according to aspects of the invention is shown schematically. A first LED 11 is connected in series with a second LED 12. The first and second LEDs 11, 12 are connected in series between supply potential V_{CC} and ground potential. In series with the first and second LEDs 11, 12 is a current source 13. The current source 13 is shown here as being a controllable current source. It is controllable in the sense that it can be switched on and off. It is optional that the current source 13 is controllable also as to the particular current that is delivered.

A controller 14 is provided. The controller has a first control output 15 that is connected to a control input of the controllable current source 13.

Connected in parallel across the first LED 11 is a first switch 18. A second switch 19 is connected in parallel across the second LED 12. The first and second switches 18, 19 are controllable switches. A second control output 16 of the controller 14 is connected to a control input of the first controllable switch 18. A third control output 17 of the controller 14 is coupled to a control input of the second switch 19.

First and second main terminals of the first switch 18 are coupled to anode and cathode electrodes respectively of the first LED 11. First and second main terminals of the second controllable switch 19 are coupled to anode and cathode electrodes respectively of the second LED 12. As such, a main electrode of the first controllable switch 18 is coupled directly to a main electrode of the second controllable switch 19. Also, the other main electrode of the first switch 18 is connected directly to supply potential V_{CC} . Furthermore, the other main electrode of the second controllable switch 19 is connected directly to an output of the controllable current source 13.

A memory 20 is included within the controller 14. The memory 20 is configured to hold calibration data that is used by the controller 14 in controlling the other components of the apparatus 10, as will now be described.

The apparatus 10 forms part of a supplementary device for attachment to a injection device. The supplementary device may be as shown and described in WO 2011/117212, the contents of which are incorporated herein by reference. Figure 4 and the corresponding description are particularly incorporated herein by reference. The parts of the apparatus 10 that are shown in Figure 1 constitute an illumination arrangement that is used to illuminate the number sleeve of the invention device 1 in WO 2011/117212, so that the OCR reader 25 is able to read the numbers present on the number sleeve at the position of the dose reading window 13.

In this particular example, the controller 14 is configured to cause the arrangement comprising the first and second LEDs 11, 12 to be illuminated for a fixed period of time. The period of time may, for instance, be equal to the period of time required by a camera of the OCR reader to capture an image of the number sleeve of the injection pen to which the apparatus 10 is coupled, allowing an optical character recognition function of the apparatus to determine a number that is shown on a number sleeve in the field of view of the camera.

To achieve illumination of the first and second LEDs 11, 12, the controller 14 is configured to provide a signal 21 on the first control output 15 to the controllable current source 13. The control signal 21 provided to the controllable current source 13 rises from a low level at time t1 to a high level and remains at the high level until a time t6, at which time it reverts to the low level. The time elapsed between t1 and t6 is equal to the duration for which the arrangement comprising the first and second LEDs 11, 12 is energised. While the control signal 21 has a high value, the controllable current source 13 is caused to draw current from supply voltage V_{CC} through the LED arrangement 11, 12 to ground potential.

In this particular example, the second LED 12 has a higher light intensity output than the first LED 11, for a given current. As such, the provision of a particular current through both of the LEDs 11, 12 for the same period of time results in more light being produced by the second LED 12 than is produced by the first LED 11. This is avoided by the arrangement of the apparatus 10, in particular by operation of the controller 14.

The controller 14 is configured to provide the first control signal 22, shown in Figure 2, on the second control output 16 to the control input of the first controllable switch 18. The first control signal 22 that is provided to the first controllable switch 18 has a low value at all times. As such, the first controllable switch 18 is caused to be open for the entire time that the apparatus 10 is operating, and at least for the duration between t1 and t6 for which the controllable current source 13 is controlled to provide current to the LED arrangement 11, 12.

However, the controller is configured to cause the second LED 12 to be provided with source current for only a portion of the period between times t1 and t6. In particular, the controller 14 is configured to provide on the third control output 17 to the control input of the second controllable switch 19 the third control signal 23, shown in Figure 2. It can be seen from Figure 2 that the third control signal 23 has a value of zero for most of the time between t1 and t6, but for some of the time the value of the control signal is high. In particular, the third control signal 23 is high for an interval between times t5 and t6, and is low for the interval between times t1 and t5. When the third control signal 23 is low, the second controllable switch 19 is open. When both the first and second controllable switches 18, 19 are open, all of the current provided by the controllable current source 13 flows through both of the first and second LEDs 11, 12. However, when the third control signal 23 is high, the second controllable switch 19 is closed. In this condition, current provided by the controllable current source 13 flows through the first LED 11 but bypasses the second LED 12 by flowing instead through the second controllable switch 19, then to the controllable current source 13. As such, for the periods where the second control signal 23 is high, the second LED 12 is not energised and does not emit light. It will be apparent that the second LED 12 does not emit light when the second controllable switch 19 is closed because when the second controllable switch is in this state the resistance of the switch is much lower than the resistance of the LED 12, and thus significant current does not flow through the second LED 12. Put another way, the voltage across the anode and cathode electrodes of the second LED 12 is insufficient for significant levels of current to flow through the second LED 12.

So, for the duration for which the controllable current source 13 is energised, by the high part of the first control signal 21, the first LED 11 is illuminated for the entire time and the second LED 12 is illuminated for only part of the time. Because the brightness of the LEDs is dependent on the current, the quantity of light that is emitted by the LEDs individually in the periods between times t1 and t6 is a function of the light intensity output of the LED when it is provided with the energising current and the period of time for which the LED is illuminated.

By providing calibration data in the memory 20 of the controller 14 such as to cause the controller 14 to close the second controllable switch 19 for the correct proportion of the time interval between t1 and t6, the overall light output of the first and second LEDs 11, 12 within the time interval t1 to t6 can be caused to be the same. In particular, by providing the calibration data stored in the memory 20 with a value such as to cause the controller 14 to provide the third control signal 23 with a duty cycle (the ratio of the duration of the low value of the signal (t5 minus t1) to the period of the signal (t6 minus t1) that is equal to the ratio of the output light intensity of the second LED 12 to the output light intensity of the first LED 11, the total light output by the first LED 11 within the time period t1 to t6 is equal to the total light output by the second LED 12 within the same time period. Rearranging this, the interval between times t1 and t5 is calculated as the time interval between t1 and t6 multiplied by the ratio of the light intensity output of the first LED 11 to the light intensity output of the second LED 12.

Figure 2 also shows an alternative way in which the controller 14 may be configured to provide substantially the same effect. In particular, the controller 14 is configured to provide a fourth control signal 24 on the third control output 17 to the control input of the second controllable switch 19, in place of the third control signal 23. The fourth control signal 24 is a pulse width modulated signal, which has plural periods in which the signal has a high value, with successive periods of high signal level separated by periods in which the control signal has a low value. With such a signal, the controller 14 is configured to provide equal distances between successive changes from low to high control signal value. The controller 14 is configured also to provide the periods of high control signal value with equal durations to one another.

In this example, the periods of low control signal value have a period between times t2 and t3. Periods of high control signal value have a duration between time intervals t3 and t4. In this particular example, there are six periods of high control signal value.

The effect of the fourth control signal 24 is the same as that of the third control signal 23 because the overall duration of the high control signal periods is equal between the two control signals 23, 24. In particular, the sum of the time durations t3 to t4 for each of the multiple high control value periods for the fourth control signal 24 equals the duration of the time period t5 to t6. As such, provision by the controller 14 of the fourth control signal 24 via the third control signal output 17 to the control input of the second controllable switch 19 causes the same overall illumination by the second LED 12, although the illumination is provided at different parts of the time period t1 to t6 than is the case for the third control signal 23.

In another alternative, pulse density modulation is used. Here, the controller 14 is configured to provide plural pulses of the same length but with an interval between successive pulses that results in the desired duty cycle. Here, the stored calibration data may be used to derive the interval between successive pulses directly.

With the above-described arrangement, each of the LEDs 11, 12 is controlled to provide a predetermined amount of light output, relative to the other LED. Advantageously, this is achieved through the use of a single controllable source of energy, here the controllable current source 13. The result is an arrangement that is considerably simpler in terms of hardware, and thus less expensive to produce, than the multiple current source arrangement that was described in the background section of this specification. Moreover, with this particular arrangement, this can be achieved without the use of a DC to DC converter that would typically be required in the arrangement in which balancing of the light outputs of the LEDs were achieved using separate current control circuits. Thus, there is a further reduction in the amount of hardware required to provide the balancing of the light outputs of the LEDs. The only additional hardware that is required in this arrangement 10, compared to the alternative, is the first and second controllable switches 18, 19.

The calibration data stored in the memory 20 may take any suitable form. It may, for instance, define the time period t1 to t5, or it may define the time period t5 to t6. It may additionally define the time period t1 to t6, although this may be known to the controller 14 in some other way. The calibration data may take a form of a simple numerical value. The numerical value may define the absolute value of time, for instance the interval t1 to t5, or it may define the ratio of the low to high value periods of the control signal, for instance by defining the ratio of t1 to t5 to t5 to t6. In order for the arrangement 10 to provide equal light output from the first and second LEDs 11, 12, the calibration data 20 is derived from the actual light intensity outputs of the first and second LEDs 11, 12. This may be achieved by measuring light intensity outputs of the LEDs 11, 12 in a calibration process during manufacturing of the apparatus 10, with the calibration data being stored in the memory 20 as part of the calibration process. In this way, correct operation of the apparatus 10 can then easily be tested by causing the controller 14 to use the calibration data stored in the memory 20 to cause the one of the LEDs 11, 12 to be switched off for some of the interval for which the other LED is illuminated, as described above. By measuring the overall light output of the LEDs 11, 12 during the time interval t1 to t6, the calibration process can ensure that the value of the calibration data stored in the memory 20 is correct so as to give rise to equal light output by the LEDs 11, 12.

Provision of the fourth control signal 24 by the controller 14 based on calibration data stored in the memory 20 is achieved in a corresponding way to that described above in relation to the third control signal 23. In particular, the calibration data may define values of intervals t2 to t3 and t3 to t4, or a ratio thereof, or it may define absolute values for the times, for instance.

It will be appreciated from the above example that the first controllable switch 18 is not operated at all. This is because the second LED 12 has a higher light intensity output that the second LED 11. In the event that the first LED 11 has the higher light intensity output, the controller 14 operates only the first controllable switch 18, and the second controllable switch 19 is never operated. If it is known or can be determined prior to assembly of the apparatus 10 which of two LEDs 11, 12 has the higher light output, the apparatus 10 may be constructed with only one controllable switch, for instance the first controllable switch 18, with the other controllable switch and the corresponding control output from the controller 14 being omitted. However, this may require either testing of LEDs 11, 12 before they are fitted onto a circuit board (or otherwise installed in the apparatus 10), or the intentional selection of different models of LED such that it is always known which has the higher light intensity output.

The controller 14 may take any suitable form. For instance, it may be a basic microcontroller.

The memory 20 may take any suitable form. It may be only a byte in size, or a small number of bytes. It may be a traditional memory such as ROM, EEPROM etc. It may alternatively be any other suitable device for storing data, such as a simple transistor circuit, a simple non-transistor circuit, a memristor arrangement, an optical or magnetic storage component, etc.

Various other alternative implementations will be apparent to the skilled person, and all such alternatives are within the scope of the invention unless excluded from the wording of the appended claims.

The controllable switches 18, 19 may take any suitable form. For instance, they may be bipolar transistors, MOSFET transistors or some other type of transistor. The first and second controllable switches 18, 19 may alternatively be electromechanical devices, such as reed switches, relays etc. They may alternatively be analogue current bypass devices, of any suitable form.

Although in the above first and second LEDs 11, 12 are described as light sources connected in series, other forms of light sources in which brightness is dependent on current may alternatively be used. This includes various photoemitters whether currently available or yet to be marketed. The light sources may operate exclusively within visible wavelengths, or may operate partly or completely into ultraviolet and/or infrared frequencies.

The controllable current source 13 may take any suitable form. The use of a current source is particularly advantageous for light sources of certain types, including LEDs, but the form may vary, for instance if there is a limited amount of voltage headroom available or if a relatively large number of light sources are connected in series.

Although in the above first and second light sources 11, 12 are shown and described, in other embodiments three, four or further light sources are connected in series between supply terminals V_{CC} and ground, in series with the controllable current source (or alternative).

In the above, the calibration data is such as to cause the LEDs to have equal light emission where they have different brightnesses. Here, the outputs of the LEDs are balanced in that the outputs are equal. It will be appreciated that the invention is applicable also where different light emission is required from different LEDs, whether or not they have the same brightness. For instance, in some embodiments the calibration data is such as to cause the light emitted by a first LED to be 80% of the light emitted from a second LED. The balance here is 5:4.

The LEDs is the above are the same type of LED and as such have the same colour characteristics. In other embodiments, different LED types are used, having different colour characteristics. By using the features described above, the arrangement can be caused to provide a required balance of colour. The balance may be equal (e.g. the same output of red and green and blue) or the balance may be unequal (e.g. a balance of 4:3 blue to green). Although in the above the apparatus 10 is said to be a supplemental or clip-on device for use with an injection pen, it will be appreciated that the invention is more broadly applicable to this and that the scope of the invention extends to any apparatus in which the relative light output of two or more light sources connected in series between supply terminals is required to be controlled.

Although the first and second controllable switches 18, 19 are open when a low level control signal is applied, the switches may alternatively be closed unless a high control signal is applied. Here, the inverse of the second to fourth control signals 22 to 24 are used to provide the effect described above.

## Claims

1. Apparatus comprising at least two light sources including:
a first light source;
a second light source connected in series with the first light source between first and second supply terminals;
a controllable switch coupled in parallel across the first light source; and
a controller,
wherein the controller is configured to selectively control the controllable switch to conduct, thereby selectively switching the first light source on and off whilst the second light source remains switched on.

2. Apparatus as claimed in claim 1, comprising a current sink connected in series with the first and second light sources between the first and second supply terminals.

3. Apparatus as claimed in either preceding claim, comprising a second controllable switch coupled in parallel across the second light source, wherein the controller is configured to selectively control the second controllable switch to conduct, thereby selectively switching the second light source on and off whilst the first light source remains switched on.

4. Apparatus as claimed in any preceding claim, wherein the at least two light sources further comprise one or more further light sources connected in series with the first and second light sources between the first and second supply terminals.

5. Apparatus as claimed in claim 4, wherein each further light source is provided with a further controllable switch coupled in parallel across the further light source, and wherein the controller is configured to selectively control the further controllable switch to conduct, thereby selectively switching the further light source on and off whilst the other light sources remains switched on.

6. Apparatus as claimed in any preceding claim, wherein the or each controllable switch is a transistor.

7. Apparatus as claimed in any of claims 1 to 5, wherein the or each controllable switch is electro-mechanical.

8. Apparatus as claimed in any of claims 1 to 5, wherein the or each controllable switch is an analogue current bypass device.

9. Apparatus as claimed in any preceding claim, wherein the controller is configured to selectively control a controllable switch to conduct by applying a pulse width modulated signal to the controllable switch.

10. Apparatus as claimed in claim 9, wherein a duty cycle of the pulse width modulated signal is derived from calibration data stored in a memory of the apparatus.

11. Apparatus as claimed in any of claims 1 to 8, wherein the controller is configured to selectively control a controllable switch to conduct by applying a signal of a specific duration to the controllable switch.

12. Apparatus as claimed in claim 11, wherein the specific duration is derived from calibration data stored in a memory of the apparatus.

13. Apparatus as claimed in any preceding claim, wherein the light sources are light-emitting diodes.

14. Apparatus as claimed in any preceding claim, wherein the light sources are the same model of device.

15. A supplemental device for mating to an injection device, the supplemental device comprising apparatus as claimed in any preceding claim, in which the light sources illuminate a number sleeve of the injection device in use.
